# EUROPÄISCHE PATENTSCHRIFT

(11) **EP 1 313 860 B1**
(45) Veröffentlichungstag und Bekanntmachung des Hinweises auf die Patenterteilung: **14.10.2009**
(21) Anmeldenummer: 01962989.8
(22) Anmeldetag: 30.08.2001
(51) Int. Cl.: C12N 15/55, C12N 9/16, C12N 15/63, C12N 1/20, C12N 1/21, C12P 41/00, C12P 7/00, C12R 1/38

(54) **BUTINOL I ESTERASE**
BUTINOL I ESTERASE
BUTINOL I ESTERASE

(30) Priorität: 31.08.2000 DE 10042892; 29.06.2001 DE 10131544
(43) Veröffentlichungstag der Anmeldung: 28.05.2003
(73) Patentinhaber: BASF SE, 67056 Ludwigshafen (DE)
(72) Erfinder: HAUER, Bernhard, 67136 Fussgönheim (DE); FRIEDRICH, Thomas, 64283 Darmstadt (DE); NÜBLING, Christoph, 67454 Hassloch (DE); STÜRMER, Rainer, 67127 Rödersheim-Gronau (DE); LADNER, Wolfgang, 67136 Fussgönheim (DE)
(74) Vertreter: Kinzebach, Werner
(86) Internationale Anmeldenummer: PCT/EP2001/010040
(87) Internationale Veröffentlichungsnummer: WO 2002/018560

(56) Entgegenhaltungen:
- EP-A- 1 069 183
- US-A- 5 518 903
- DATABASE WPI Section Ch, Week 198827 Derwent Publications Ltd., London, GB; Class B05, AN 1988-186422 XP002188822 & JP 63 123399 A (CHISSO CORP), 27 May 1988 (1988-05-27)
- DATABASE WPI Section Ch, Week 198243 Derwent Publications Ltd., London, GB; Class B05, AN 1982-91810E XP002188823 & JP 57 152894 A (UBE IND LTD), 21 September 1982 (1982-09-21)
- DATABASE WPI Section Ch, Week 198834 Derwent Publications Ltd., London, GB; Class B05, AN 1988-238009 XP002188824 & JP 63 169997 A (CHISSO CORP), 13 July 1988 (1988-07-13)
- DATABASE SWALL [Online] EBI; 1 November 1998 (1998-11-01) "Lactone-specific esterase ESTF1 from P. fluorescens" XP002188820
- ROGALSKA, E. ET AL.: "Stereoselective Hydrolysis of Triglycerides by Animal and Microbial Lipases" CHIRALITY, vol. 5, 1993, pages 24-30, XP001053083
- DATABASE EMBL [Online] EBI; 21 September 1994 (1994-09-21) "A. vinelandii NADPH : ferredoxin reductase gene." XP002188821
- BAUMANN M ET AL: "Rapid screening of hydrolases for the enantioselective conversion of 'difficult-to-resolve' substrates" TETRAHEDRON: ASYMMETRY, ELSEVIER SCIENCE PUBLISHERS, AMSTERDAM, NL, vol. 11, no. 23, 1 December 2000 (2000-12-01), pages 4781-4790, XP004313852 ISSN: 0957-4166
- KREBSFAENGER N ET AL.: "Characterization and enantioselectivity of a recombinant esterase from Pseudomonas fluorescens" ENZYME AND MICROBIAL TECHNOLOGY, Bd. 22, 15. Mai 1998 (1998-05-15), Seiten 641-646,
- DATABASE UniProt [Online] 15. Dezember 1998 (1998-12-15), gefunden im EBI Database accession no. UNIPROT:FENR_AZOVI Q44532

### SEQUENZ PROTOKOLL

<110> BASF Aktiengesellschaft
<120> Butinol I Esterase
<130> M/42107 Butinol I Esterase
<140>
   <141>
<160> 6
<170> PatentIn Ver. 2.1
<210> 1
   <211> 1530
   <212> DNA
   <213> Pseudomonas glumae LU 2023
<220>
   <221> CDS
   <222> (1)..(1530)
<400> 1
   atg atc gtc caa ctg atc gcc atc gtg gtc gcc ctc tac gcc gtg ctg    48
   Met Ile Val Gln Leu Ile Ala Ile Val Val Ala Leu Tyr Ala Val Leu
     1               5                  10                  15
   ttc gcg ttc acg ctg ttc acc gcg cat cag gtg cgc cgc cgc ttt ccg    96
   Phe Ala Phe Thr Leu Phe Thr Ala His Gln Val Arg Arg Arg Phe Pro
                20                  25                  30
   ccc gag ggc aag ttc gtc gag atc gac ggc gac cgc ctg cat tat gtc    144
   Pro Glu Gly Lys Phe Val Glu Ile Asp Gly Asp Arg Leu His Tyr Val
            35                  40                  45
   gac tac ggc agc ggg ccg ccg atc gtg atg gtg cat ggc ctg tgc ggg    192
   Asp Tyr Gly Ser Gly Pro Pro Ile Val Met Val His Gly Leu Cys Gly
        50                  55                  60
   cag ctg ctg aac ttc gcc tac ctc gat ctg gcg cgg ctc gcg cag tcg    240
   Gln Leu Leu Asn Phe Ala Tyr Leu Asp Leu Ala Arg Leu Ala Gln Ser
    65                  70                  75                  80
   cat cgc gtg atc ctc gtc gat cgg gcc ggc tcg gga cgc tcg acg cgc    288
   His Arg Val Ile Leu Val Asp Arg Ala Gly Ser Gly Arg Ser Thr Arg
                    85                  90                  95
   ggc ccc gcc tcg cgc gcg aac gtc tat gcg cag gcg cgc ggc atc gcc     336
   Gly Pro Ala Ser Arg Ala Asn Val Tyr Ala Gln Ala Arg Gly Ile Ala
               100                 105                 110
   cgc ttc atc gag acg ctc ggc ctg gag cgg ccg gtg ctg gtg ggc cat     384
   Arg Phe Ile Glu Thr Leu Gly Leu Glu Arg Pro Val Leu Val Gly His
           115                 120                 125
   tcg ctc ggc ggc gcg atc gcg ctc gcg gtc ggc ctg gac tac ccc gag     432
   Ser Leu Gly Gly Ala Ile Ala Leu Ala Val Gly Leu Asp Tyr Pro Glu
       130                 135                 140
   cgc gtg agc cgc atc gcg ctg atc gcg ccg ctc acg cac acc gag acc     480
   Arg Val Ser Arg Ile Ala Leu Ile Ala Pro Leu Thr His Thr Glu Thr
   145                 150                 155                 160
   gag ccg ccc aag gcg ttc cgc ggg ctc gcg ctg cgc ccg gcg gcg ctg     528
   Glu Pro Pro Lys Ala Phe Arg Gly Leu Ala Leu Arg Pro Ala Ala Leu
                   165                 170                 175
   cgc cgc ttc gcg tcg ctg acg atg ggc atc ccg atc atg att ctg caa     576
   Arg Arg Phe Ala Ser Leu Thr Met Gly Ile Pro Ile Met Ile Leu Gln
               180                 185                 190
   agc cgc aag gcg atc gac gcg atc ttc gcg ccg gag ccg gtg ccg cgc     624
   Ser Arg Lys Ala Ile Asp Ala Ile Phe Ala Pro Glu Pro Val Pro Arg
           195                 200                 205
   gat ttc ccg ctg aag ggc ggc ggc atg atg ggg ctg cgg ccc gag gcg     672
   Asp Phe Pro Leu Lys Gly Gly Gly Met Met Gly Leu Arg Pro Glu Ala
       210                 215                 220
   ttc tac gcg gcg tcg tcg gac ctg gtc gcc gcg ccc gag gac ctg ccc     720
   Phe Tyr Ala Ala Ser Ser Asp Leu Val Ala Ala Pro Glu Asp Leu Pro
   225                 230                 235                 240
   gac atg gag cgc cgc tac ccg acg ctg ggc gtg ccg gtc agc atg ctg     768
   Asp Met Glu Arg Arg Tyr Pro Thr Leu Gly Val Pro Val Ser Met Leu
                   245                 250                 255
   tac ggg cgc cag gac gcg atc ctc gat ttc cac aag cat ggc gag ggg     816
   Tyr Gly Arg Gln Asp Ala Ile Leu Asp Phe His Lys His Gly Glu Gly
               260                 265                 270
   ctc aag cgc aag ctc gac ggc gtc gag ctg agc gcc gtc gag ggc ggg     864
   Leu Lys Arg Lys Leu Asp Gly Val Glu Leu Ser Ala Val Glu Gly Gly
           275                 280                 285
   cac atg ctg ccc gtg acg cag ccg gcc gcc acc acc gac tgg ctc ctc     912
   His Met Leu Pro Val Thr Gln Pro Ala Ala Thr Thr Asp Trp Leu Leu
       290                 295                 300
   gcg gtg gcc gcg gcg gcg aac gcg gcg gcg cag cac gat gcg gcg cgg     960
   Ala Val Ala Ala Ala Ala Asn Ala Ala Ala Gln His Asp Ala Ala Arg
   305                 310                 315                 320
   ccg gat ccg gca ccg tcc gag gtc acg cag gcc ggc gcg ctg cag cat     1008
   Pro Asp Pro Ala Pro Ser Glu Val Thr Gln Ala Gly Ala Leu Gln His
                   325                 330                 335
   ctg aag gtc ggc gac aac gtg ctg atc ggc aag aag ccc acc ggc acg     1056
   Leu Lys Val Gly Asp Asn Val Leu Ile Gly Lys Lys Pro Thr Gly Thr
               340                 345                 350
   ctg gtg gcc gac aac ctg ctg ccg ggc aag acc ctg tgg ctg ctg tcg     1104
   Leu Val Ala Asp Asn Leu Leu Pro Gly Lys Thr Leu Trp Leu Leu Ser
           355                 360                 365
   acc ggc acg ggt ctc gcg ccg ttc atg tcg atc atc cgc gat ccg gac     1152
   Thr Gly Thr Gly Leu Ala Pro Phe Met Ser Ile Ile Arg Asp Pro Asp
       370                 375                 380
   atc tac gaa cgc tac gag aag gtg gtg ctc acg cac acc tgc cgc ctg     1200
   Ile Tyr Glu Arg Tyr Glu Lys Val Val Leu Thr His Thr Cys Arg Leu
   385                 390                 395                 400
   aag ggc gag ctc gcg tac atg gac ttc atc aag cac gac ctg ccg ggc     1248
   Lys Gly Glu Leu Ala Tyr Met Asp Phe Ile Lys His Asp Leu Pro Gly
                   405                 410                 415
   cat gag tac ctc ggc gac atc atc aag gaa aag ctg atc tac tac ccg     1296
   His Glu Tyr Leu Gly Asp Ile Ile Lys Glu Lys Leu Ile Tyr Tyr Pro
               420                 425                 430
   acc gtc acg cgc gaa gcg ttc gac aac gag ggg cgg atc acc gac ctg     1344
   Thr Val Thr Arg Glu Ala Phe Asp Asn Glu Gly Arg Ile Thr Asp Leu
           435                 440                 445
   atc tcg acg ggc aag ctg ttc acc gat ctc gac gtc ccg ccg ttc tcg     1392
   Ile Ser Thr Gly Lys Leu Phe Thr Asp Leu Asp Val Pro Pro Phe Ser
       450                 455                 460
   ccc gag aac gac cgc gtg atg ctg tgc ggc agc acc gcg atg ctg aag     1440
   Pro Glu Asn Asp Arg Val Met Leu Cys Gly Ser Thr Ala Met Leu Lys
   465                 470                 475                 480
   gac acc acc gac ctg ctc aag cag gcc ggc ctc gtc gaa ggc aag aac    1488
   Asp Thr Thr Asp Leu Leu Lys Gln Ala Gly Leu Val Glu Gly Lys Asn
                   485                 490                 495
   agc gcg ccg ggc cac tat gtg atc gaa cgc gca ttt gtc gac            1530
   Ser Ala Pro Gly His Tyr Val Ile Glu Arg Ala Phe Val Asp
               500                 505                 510
<210> 2
   <211> 510
   <212> PRT
   <213> Pseudomonas glumae LU 2023
<400> 2
   Met Ile Val Gln Leu Ile Ala Ile Val Val Ala Leu Tyr Ala Val Leu
    1               5                  10                  15
   Phe Ala Phe Thr Leu Phe Thr Ala His Gln Val Arg Arg Arg Phe Pro
               20                  25                  30
   Pro Glu Gly Lys Phe Val Glu Ile Asp Gly Asp Arg Leu His Tyr Val
           35                  40                  45
   Asp Tyr Gly Ser Gly Pro Pro Ile Val Met Val His Gly Leu Cys Gly
       50                  55                  60
   Gln Leu Leu Asn Phe Ala Tyr Leu Asp Leu Ala Arg Leu Ala Gln Ser
   65                  70                  75                  80
   His Arg Val Ile Leu Val Asp Arg Ala Gly Ser Gly Arg Ser Thr Arg
                   85                  90                  95
   Gly Pro Ala Ser Arg Ala Asn Val Tyr Ala Gln Ala Arg Gly Ile Ala
               100                 105                 110
   Arg Phe Ile Glu Thr Leu Gly Leu Glu Arg Pro Val Leu Val Gly His
           115                 120                 125
   Ser Leu Gly Gly Ala Ile Ala Leu Ala Val Gly Leu Asp Tyr Pro Glu
       130                 135                 140
   Arg Val Ser Arg Ile Ala Leu Ile Ala Pro Leu Thr His Thr Glu Thr
   145                 150                 155                 160
   Glu Pro Pro Lys Ala Phe Arg Gly Leu Ala Leu Arg Pro Ala Ala Leu
                   165                 170                 175
   Arg Arg Phe Ala Ser Leu Thr Met Gly Ile Pro Ile Met Ile Leu Gln
   180 185 190
   Ser Arg Lys Ala Ile Asp Ala Ile Phe Ala Pro Glu Pro Val Pro Arg
   195 200 205
   Asp Phe Pro Leu Lys Gly Gly Gly Met Met Gly Leu Arg Pro Glu Ala
   210 215 220
   Phe Tyr Ala Ala Ser Ser Asp Leu Val Ala Ala Pro Glu Asp Leu Pro
   225 230 235 240
   Asp Met Glu Arg Arg Tyr Pro Thr Leu Gly Val Pro Val Ser Met Leu
   245 250 255
   Tyr Gly Arg Gln Asp Ala Ile Leu Asp Phe His Lys His Gly Glu Gly
   260 265 270
   Leu Lys Arg Lys Leu Asp Gly Val Glu Leu Ser Ala Val Glu Gly Gly
   275 280 285
   His Met Leu Pro Val Thr Gln Pro Ala Ala Thr Thr Asp Trp Leu Leu
   290 295 300
   Ala Val Ala Ala Ala Ala Asn Ala Ala Ala Gln His Asp Ala Ala Arg
   305 310 315 320
   Pro Asp Pro Ala Pro Ser Glu Val Thr Gln Ala Gly Ala Leu Gln His
   325 330 335
   Leu Lys Val Gly Asp Asn Val Leu Ile Gly Lys Lys Pro Thr Gly Thr
   340 345 350
   Leu Val Ala Asp Asn Leu Leu Pro Gly Lys Thr Leu Trp Leu Leu Ser
   355 360 365
   Thr Gly Thr Gly Leu Ala Pro Phe Met Ser Ile Ile Arg Asp Pro Asp
   370 375 380
   Ile Tyr Glu Arg Tyr Glu Lys Val Val Leu Thr His Thr Cys Arg Leu
   385 390 395 400
   Lys Gly Glu Leu Ala Tyr Met Asp Phe Ile Lys His Asp Leu Pro Gly
   405 410 415
   His Glu Tyr Leu Gly Asp Ile Ile Lys Glu Lys Leu Ile Tyr Tyr Pro
   420 425 430
   Thr Val Thr Arg Glu Ala Phe Asp Asn Glu Gly Arg Ile Thr Asp Leu
   435 440 445
   Ile Ser Thr Gly Lys Leu Phe Thr Asp Leu Asp Val Pro Pro Phe Ser
   450 455 460
   Pro Glu Asn Asp Arg Val Met Leu Cys Gly Ser Thr Ala Met Leu Lys
   465 470 475 480
   Asp Thr Thr Asp Leu Leu Lys Gln Ala Gly Leu Val Glu Gly Lys Asn
   485 490 495
   Ser Ala Pro Gly His Tyr Val Ile Glu Arg Ala Phe Val Asp
   500 505 510
<210> 3
   <211> 32
   <212> PRT
   <213> Pseudomonas glumae Lu2023
<400> 3
   Phe Ile Glu Thr Leu Gly Leu Glu Arg Pro Val Leu Val Gly His Ser
   1 5 10 15
   Leu Gly Gly Ala Ile Ala Leu Ala Val Gly Leu Asp Tyr Pro Glu Arg
   20 25 30
<210> 4
   <211> 14
   <212> PRT
   <213> Pseudomonas glumae Lu2023
<400> 4
   Ile Ala Leu Ile Ala Pro Leu Thr His Thr Glu Thr Glu Pro
   1 5 10
<210> 5
   <211> 20
   <212> PRT
   <213> Pseudomonas glumae Lu2023
<400> 5
   Gly Gly Gly Met Met Gly Leu Arg Pro Glu Ala Phe Tyr Ala Ala Ser
   1 5 10 15
   Ser Asp Leu Val
   20
<210> 6
   <211> 11
   <212> PRT
   <213> Pseudomonas glumae Lu2023
<400> 6
   Ala Ile Asp Ala Ile Phe Ala Pro Glu Pro Val
   1 5 10

## Beschreibung

Die Erfindung betrifft neuartige Proteine mit Butinol I Esterase-Aktivität aus Pseudomonas glumae, dafür kodierende Nukleinsäuresequenzen, Expressionskassetten, Vektoren und rekombinante Mikroorganismen; Verfahren zur Herstellung dieser Proteine und deren Verwendung zur enzymatischen, insbesondere enantioselektiven enzymatischen esterhydrolyse oder Umesterung organischer Ester.

Esterasen und Lipasen gehören zu den Hydrolasen, die zur Synthese optisch aktiver, organischer Verbindungen in technischen Prozessen eingesetzt werden können und sich durch hohe Substratspezifität auszeichnen. Sie können in einem den Serinproteasen ähnlichen Mechanismus Acylgruppen auf Nukleophile, wie z.B. Carbonylgruppen, übertragen oder Esterbindungen hydrolytisch spalten. Den Esterasen, Lipasen und Serinproteasen ist die katalytische Triade gemeinsam, ein Sequenzmotiv aus den Aminosäuren Ser, His und Asp, wobei das aktive Ser das Carbonylkohlenstoffatom nukleophil angreift und es unter Beteiligung der anderen beiden Aminosäuren zu einer Ladungsverteilung kommt. Esterasen und Lipasen können Acylgruppen auch auf andere Nukleophile, wie Thiogruppen eines Thioethers oder aktivierte Amine, übertragen.

Lipasen hydrolysieren langkettige Glycerinester und zeichnen sich durch Grenzflächenaktivierung aus, d.h. dass das aktive Zentrum nur in Gegenwart des Lipidsubstrats zugänglich wird. Lipasen sind in nicht-wässrigen organischen Lösungsmitteln stabil und werden in zahlreichen technischen Prozessen zur kinetischen Racematspaltung eingesetzt, d.h. ein Enantiomer wird wesentlich schneller als das andere umgesetzt. Dieses läßt sich nachfolgend aufgrund unterschiedlicher physikalischer und chemischer Eigenschaften aus der Reaktionslösung gewinnen.

Nakamura (Nakamura, K. et al., Tetrahedron; Asymmetry 9, (1999), 4429-4439) beschreibt die Racematspaltung von 1-Alkyn-3-ol durch Umesterung mit Hilfe käuflich erhältlicher Lipasen (Amano AK, AH und PS; Amano Pharmaceuticals Co. Ltd.) in hydrophoben Lösungsmitteln, wobei die Enantioselektivität mit der Kettenlänge des Acyldonors steigt und sterisch große Reste (Chloracetat, Vinylbenzoat) die Reaktion negativ beeinflussen. Yang (Yang, H. et al., J. Org. Chem. 64, (1999), 1709-1712) beschreibt die enantioselektive Herstellung optisch aktiver Säuren durch Umesterung mit Vinylestern unter Verwendung der Lipase B aus Candida antarctica als Katalysator. Ethylester führen hier zu einer deutlich geringeren Reaktionsgeschwindigkeit und Selektivität. Eine aus Burkholderia plantarii (Pseudomonas plantarii oder glumae) DSM 6535 isolierte Lipase wird zur enantioselektiven Acylierung racemischer Amine mit Hilfe von Ethylmethoxyacetat eingesetzt (Balkenhohl, F. et al., J. prakt. Chem. 339, (1997), 381-384).

Esterasen katalysieren enantioselektiv die Bildung und Auflösung von Esterbindungen (Hin- und Rückreaktion). Bei der Umesterung zur Gewinnung optisch aktiver Alkohole werden vorzugsweise Vinylester eingesetzt, da die Alkoholfunktion des Esters nach der Umsetzung durch Tautomerisierung zu Aldehyd oder Keton nicht mehr zur Verfügung steht und damit die Rückreaktion vermieden werden kann. Esterasen sind im Gegensatz zu Lipasen nicht grenzflächenaktiviert und setzen auch organische Verbindungen kürzerer Kettenlänge um. Aus verschiedenen Organismen wurden Esterasen unterschiedlicher Substratspezifität isoliert.

So findet die Esterase aus Pseudocardia thermophila FERM-BP-6275 Verwendung bei der Hydrolyse optisch aktiver Chromanessigsäureester (EP-A-0 892 044).

Eine Esterase aus Bacillus acidocaldarius hydrolysiert Ester eines engen Substratspektrums und mit niedriger Enantioselektivität (Manco, G. et al., Biochem. J. 332, (1998), 203-212).

Acylase 1 aus Aspergillus wird zur Gewinnung sekundärer Alkohole durch Umesterung mit vinylestern in organischen unpolaren Lösungsmitteln verwendet, wobei bevorzugt sekundäre Alkohole mit kurzen Seitenketten umgesetzt werden (Faraldos, J. et al., Synlett 4, (1997), 367-370). Aus Pseudomonas fluorescens DSM 50 106 wurde eine Membran-gebundene Lacton-spezifische Esterase kloniert (Khalameyzer, V. et al., Appl. and Environ. Microbiol. 65(2), (1999), 477-482), und aus der Q-Mutante von E. coli eine Acetylesterase (Peist, R. et al., J. Bacteriol. 179, (1997), 7679-7686). Jedoch wurden Enantioselektivität und Substratspezifität dieser beiden Esterasen nicht näher untersucht wurden. Rhodococcus sp. NCBM 11216 exprimiert 4 Esterasen, RR1 bis RR4, die unterschiedliche Spezifität aufweisen. Bei der Estersynthese aus Naphthol und einer Säure bevorzugen RR1 und RR2 Säuren mit kurzen Kohlenstoffketten, während RR3 und RR4 spezifisch Säuren mit längeren Kohlenstoffketten und sterisch größeren Resten umsetzen (Gudelj, M. et al., J. Mol. Cat. B, Enzymatic 5, (1998), 261-266).

Krebsfänger, N. et al., beschreiben in Enzyme Microb. Technol., 1998, Bd. 22, 641-646 die Isolierung einer Esterase aus Pseudomonas fluorescens.

Zur Herstellung kleiner organischer Moleküle, wie optisch aktiver Alkohole, Säuren oder Ester mit kurzen Kohlenstoffketten, stehen jedoch keine Esterasen zur Verfügung, die ein breites Substratspektrum besitzen, eine hohe Enantioselektivität aufweisen und in technischen Prozessen eingesetzt werden können. Aufgabe der vorliegenden Erfindung ist die Bereitstellung von Esterasen, welche wenigstens eine der oben genannten Eigenschaften aufweisen.

Diese Aufgabe konnte überraschenderweise durch die Bereitstellung eines Proteins mit Butinol I Esterase-Aktivität nach Anspruch-, welches wenigstens eine Aminosäure-Teilsequenz gemäß SEQ ID NO:3, 4, 5 oder 6:
a) FIETLGLERPVLVGHSLGGAIALAVGLDYPER (SEQ ID NO:3),
b) IALIAPLTHTETEP (SEQ ID NO:4),
c) GGGMMGLRPEAFYAASSDLV (SEQ ID NO:5)
d) AIDAIFAPEPV (SEQ ID NO:6)
   (jeweils angegeben im Aminosäure-Einbuchstabencode, wobei die jeweils erste Aminosäure dem jeweiligen aminoterminalen Ende entspricht)
   umfasst,

Insbesondere wurde die Aufgabe durch Bereitstellung einer Butinol I Esterase nach Anspruch 2 gelößt, die eine Aminosäuresequenz gemäß SEQ ID NO:2 25 umfasst oder von einer Nukeinsäuresequenz gemäß SEQ ID NO: 1 kodiert wird.

Die oben genannten Proteine werden im Folgenden der Einfachheit halber auch als Butinol I Esterasen bezeichnet.

"Funktionale Äquivalente" oder Analoga der konkret offenbarten Polypeptide oder Proteine sind im Rahmen der vorliegenden Erfindung davon verschiedene Polypeptide oder Proteine, welche weiterhin die in den Patentansprüchen definierte Ezymaktivität sowie die in der Patentansprüchen definierten Strukturmerkmale besitzen.

Solche "funktionalen Äquivalenten" sind erfindungsgemäß insbesondere Mutanten, welche in wenigstens einer Sequenzpositionen von SEQ ID NO:2 eine andere als die konkret genannte Aminosäure aufweisen aber trotzdem wenigstens eine erfindungsgemäße biologische Aktivität besitzen. "Funktionale Äquivalente" umfassen somit die durch eine oder mehrere Aminosäure-Additionen, -Substituenten, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Solche "funktionalen Äquivalente" umfassen natürlich auch Polypeptide welche aus anderen Organismen, zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

Solche "funktionalen Äquivalente" umfassen ebenfalls Fragmente des erfindungsgemäßen Polypeptids gemäss SEQ ID NO:2, welche weiterhin die beanspruchten Struktur- und Aktivitätsmerkmale aufweisen.

Solche "funktionalen Äquivalente" sind außerdem Fusionsproteine, die eines der hierin beschriebenden Proteine und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitiernde Beispiele für derartige heterologe Sequenzen sind z.B. Signalpeptide, Enzyme, Immunoglobuline, Oberflächenantigene, Rezeptoren oder Rezeptorliganden.

Erfindungsgemäße "funktionale Äquivalente" sind Homologe zu den konkret offenbarten Polypeptiden oder Proteinen. Diese besitzen wenigstens 60 %, vorzugsweise wenigstens 75% ins besondere wenigsten 85 %, wie z.B. 90%, 95% oder 99%, Homologie zu der konkret offenbarten Sequenz gemäß SEQ ID NO:2, berechnet nach dem Algorithmus von Pearson und Lipman, Proc. Natl. Acad, Sci. (USA) 85(8), 1988, 2444-2448.

Homologe der erfindungsgemäßen Proteine oder Polypeptide können durch Mutagenese erzeugt werden, z.B. durch Punktmutation oder Verkürzung des Proteins. Der Begriff "Homolog", wie er hier verwendet wird, betrifft eine variante Form des Proteins, die als Agonist oder Antagonist der Protein-Aktivität wirkt.

Homologe des erfindungsgemäßen Proteine können durch Screening kombinatorischer Banken von Mutanten, wie z.B. verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen codieren. Verfahren zur Synthese degenerierter Oligonukleotide sind dem Fachmann bekannt (Z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Zusätzlich können Banken von Fragmenten des Protein-Codons verwendet werden, um eine variegierte Population von Protein-Fragmenten zum Screening und zur anschließenden Selektion von Homologen eines erfindungsgemäßen Proteins zu erzeugen. Bei einer Ausführungsform kann eine Bank von kodierenden Sequenzfragmenten durch Behandeln eines doppelsträngigen PCR-Fragmentes einer kodierenden Sequenz mit einer Nuklease unter Bedingungen, unter denen ein Nicking nur etwa einmal pro Molekül erfolgt, Denaturieren der doppelsträngigen DNA, Renaturieren der DNA unter Bildung doppelsträngiger DNA, die Sense-/Antisense-Paare von verschiedenen genickten Produkten umfassen kann, Entfernen einzelsträngiger Abschnitte aus neu gebildeten Duplices durch Behandlung mit Sl-Nuclease und Ligieren der resultierenden Fragmentbank in einen Expressionsvektor erzeugt werden. Durch dieses Verfahren kann eine Expressionsbank hergeleitet werden, die N-terminale, C-terminale und interne Fragmente mit verschiedenen Größen des erfidungsgemäßen Proteins kodiert.

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese von erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen codiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Bevorzugte erfindungsgemäße funktionale Äquivalente sind in Anspruch 2 definiert und weisen eine von SEQ ID NO: 2 in mindestens einer Position abweichende Sequenz auf, wobei diese Veränderung in der Sequenz die Esteraseaktivität vorzugsweise nur unwesentlich, d.h nicht um mehr als etwa ± 90, insbesondere ± 50 % oder nicht mehr als ± 30 % verändert. Diese Veränderung kann unter Verwendung eines Referenzsubstrates, wie z.B. Butinol-butyrat unter standardisierten Bedingungen (wie z.B. 20mM Substrat, 10 mM Phosphatpuffer, pH 7,4 T = 20 °C) bestimmt werden.

Die erfindungsgemäßen Butinol I Esterasen weisen vorzugsweise ein Molekulargewicht von etwa 40 bis 42 kDa, insbesondere etwa 41,3 kDa, bestimmt durch SDS-Gelelektrophorese auf. Sie sind insbesondere aus Pseudomonas glumae Lu 2023 der Hinterlegungsnummer DSM 13176 erhältlich. Weitere Stammvarianten sind z.B. ausgehend von Pseudomonas glumae Lu 8093 durch Selektion, wie beispielsweise durch Kultur auf Minimalmedium-Platten, mit Ethylphenylacetat als einziger Kohlenstoffquelle, zugänglich; diese sind aber nicht Gegenstand der vorliergenden Erfindung.

Die Erfindung umfasst auch Polynukleotide gemäss der Definition in den beiliegenden Patentansprüchen.

Gegenstand der Erfindung sind insbesondere Nukleinsäuresequenzen (einzel- und doppelsträngige DNA- und RNA-Sequenzen, wie z.B. cDNA und mRNA), kodierend für eines der obigen Polypeptide oder Proteine.

Die Erfindung betrifft sowohl isolierte Nukleinsäuremoleküle, welche für erfindungsgemäße Polypeptide bzw. Proteine oder biologisch aktive Abschnitte davon kodieren, sowie Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies läßt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die Erfindung umfasst weiterhin die zu den konkret beschriebenen Nukleotidsequenzen komplementären Nukleinsäuremoleküle oder einen Abschnitt davon, gemäß der Definition in Anspruch 6.

Solche erfindungsgemäße Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Solche erfindungsgemäße Nukleinsäuresequenzen sind abgeleitet von SEQ ID NO:1 und unterscheiden sich davon durch Addition, Substitution, Insertion oder Deletion einzelner oder mehrerer Nukleotide, kodieren aber weiterhin für Proteine mit erfindungsgemäße Butinol-I-Esteraseaätivität im oben genannten Schwankungsgereich der enzymatischen Aktivität.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon. Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Erfindungsgemäße Polynukleotide lassen sich bei Durchmusterung von genomischen oder cDNA-Banken auffinden und gegebenenfalls daraus mit geeigneten Primern mittels PCR vermehren und anschließend beispielsweise mit geeigneten Sonden isolieren. Eine weitere Möglichkeit bietet die Transformation geeigneter Mikroorganismen mit erfindungsgemäßen Polynukleotiden oder Vektoren, die Vermehrung der Mikroorganismen und damit der Polynukleotide und deren anschließende Isolierung. Darüber hinaus können erfindungsgemäße Polynukleotide auch auf chemischem Wege synthetisiert werden.

Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z.B. in Ausubel et al., Current Protocols in Molecular Biology, John Wiley & Sons, N.Y. (1989), 6.3.1-6.3.6. beschrieben.

Gegenstand der Erfindung sind auch Expressionskassetten, die wenigstens ein erfindungsgemäßes Polynukleotid umfassen, das mit regulatorischen Nukleinsäuresequenzen operativ verknüpft ist. Vorzugsweise liegt 5'-stromaufwärts vom erfindungsgemäßen Polynukleotid eine Promotorsequenz und ermöglicht auf diese Weise eine kontrollierte Expression der Butinol I Esterase. Besonders bevorzugt liegt 3'-stromabwärts vom erfindungsgemäßen Polynukleotid eine Terminatorsequenz sowie gegebenenfalls weitere übliche regulatorische Elemente, und zwar jeweils operativ verknüpft mit der die Butinol I Esterase kodierenden Sequenz. Unter einer operativen Verknüpfung versteht man die sequentielle Anordnung von Promotor, kodierender Sequenz, Terminator und gegebenenfalls weiterer regulatorische Elemente derart, dass jedes der regulatorischen Elemente seine Funktion vor, während oder nach Expression der kodierenden Sequenz bestimmungsgemäß erfüllen kann. Beispiele für weitere operativ verknüpfbare Sequenzen sind Targeting-Sequenzen sowie Translationsverstärker, Enhancer, Polyadenylierungssignale und dergleichen. Weitere brauchbare regulatorische Elemente umfassen selektierbare Marker, Reportergene, Amplifikationssignale, Replikationsursprünge und dergleichen.

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Die Expressionskassette kann aber auch einfacher aufgebaut sein, d.h. es werden keine zusätzlichen Regulationssignale vor das Strukturgen insertiert und der natürliche Promotor mit seiner Regulation wird nicht entfernt. Stattdessen wird die natürliche Regulationssequenz so mutiert, dass keine Regulation mehr erfolgt und die Genexpression gesteigert oder verringert wird. Die Nukleinsäuresequenzen können in einer oder mehreren Kopien in der Expressionskassette enthalten sein.

Beispiele für brauchbare Promotoren sind: cos-, tac-, trp-, tet-, trp-tet-, lpp-, lac-, lpp-lac-, lacIq-, T7-, T5-, T3-, gal-, trc-, ara-, SP6-, 1-PR- oder 1-PL-Promotor, die vorteilhafterweise in gram-negativen Bakterien Anwendung finden; sowie die gram-positiven Promotoren amy und SPO2, die Hefepromotoren ADC1, MFa , AC, P-60, CYC1, GAPDH oder die Pflanzenpromotoren CaMV/35S, SSU, OCS, lib4, usp, STLS1, B33, nos oder der Ubiquitin- oder Phaseolin-Promotor. Besonders bevorzugt ist die Verwendung induzierbarer Promotoren, wie z.B. licht- und insbesondere temperaturinduzierbarer Promotoren, wie der PᵣPₗ-Promotor.

Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und die Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA erhöht wird.

Die Herstellung einer erfindungsgemäßen Expressionskassette erfolgt durch Fusion eines geeigneten Promotors mit einem geeigneten die Butinol I Esterase kodierenden Polynukleotid, sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Gegenstand der Erfindung sind auch rekombinante Vektoren zur Transformation von eukaryontischen und prokaryontischen Wirten, die ein erfindungsgemäßes Polynukleotid oder eine erfindungsgemäße Expressionskassette tragen. Diese Vektoren erlauben in einem geeigneten Wirtsorganismus eine Expression der Butinol I Esterase. Vektoren sind dem Fachmann wohl bekannt und können beispielsweise aus "Cloning Vector" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion rekombinanter Esterase eingesetzt werden können. Vorteilhafterweise werden die oben beschriebenen erfindungsgemäßen rekombinanten Expressionskassetten als Teil eines Expressionsvektors in ein geeignetes Wirtssystem eingebracht und exprimiert. Dabei werden vorzugsweise dem Fachmann bekannte geläufige Klonierungs- und Transfektionsmethoden verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, beschrieben.

Als Wirtsorganismen zur Transformation mit erfindungsgemäßen Vektoren sind prinzipiell alle Organismen geeignet, die eine Expression der erfindungsgemäßen Polynukleotide, ihrer Allelvarianten, ihrer funktionellen Äquivalente oder Derivate ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z.B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9 oder CHO-Zellen. Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Organismen, die ein entsprechendes Antibiotikaresistenzgen tragen, lassen sich durch entsprechende Antibiotika enthaltende Medien oder Nährböden selektieren. Markerproteine, die an der Zelloberfläche präsentiert werden, können zur Selektion mittels Affinitätschromatographie genutzt werden.

Gegenstand der Erfindung sind somit auch Mikroorganismen, die einen erfindungsgemäßen Vektor tragen sowie die Pseudomonas glumae-Mutante, Lu 2023, mit der Hinterlegungsnummer DSM 13176, die die Butinol I Esterase endogen exprimiert.

Die erfindungsgemäßen Butinol I Esterasen sind insbesondere **dadurch gekennzeichnet, dass** sie neben ihrer Befähigung zun enantioselektiven Hydrolyse von Carbonsäurebintinylestern zusätzlich wenigstens eine der folgenden Reaktionen katalysieren:
a) enantioselektive Hydrolyse optisch aktiver Ester der Formel I

   R¹-COO-R² (I),

   worin R¹ für geradkettiges oder verzweigtes, gegebenenfalls ein- oder mehrfach substituiertes C₁-C₁₀-Alkyl, C₂-C₁₀-Alkenyl, C₂-C₁₀-Alkinyl und R² für geradkettiges oder verzweigtes, gegebenenfalls ein- oder mehrfach substituiertes C₁-C₁₀-Alkyl, C₂-C₁₀-Alkenyl, C₂-C₁₀-Alkinyl, C₇-C₁₅-Aralkyl oder für einen ein- oder mehrkernigen, gegebenenfalls ein- oder mehrfach substituierten aromatischen Rest steht,
   R¹ und/oder R² wenigstens ein asymmetrisches Kohlenstoffatom umfasst,
   wobei besonders bevorzugt entweder das mit dem Kohlenstoffatom oder das mit dem Sauerstoffatom der Esterbindung verbundene Kohlenstoffatom aus R¹ bzw. R² ein asymmetrisches Kohlenstoffatom ist; und
b) enantioselektive Umesterung eines Esters der Formel I mit einem optisch aktiven Alkohol der Formel II

   R²-OH (II),

   worin R² eine der obenstehenden Bedeutungen besitzt, und gegebenenfalls wenigstens ein asymmetrisches Kohlenstoffatom aufweist,
wobei besonders bevorzugt, das Kohlenstoffatom, welches die OH-Gruppe trägt, ein asymmetrisches Kohlenstoffatom ist.

Gegenstand der Erfindung sind auch Verfahren zur enantioselektiven Esterhydrolyse unter Verwendung einer in den Patentansprüchen definierten Butinol I Esterase, wobei die Butinol I Esterase mit einem Stereoisomerengemisch eines optisch aktiven Esters der Formel I in Kontakt gebracht wird und die bei der stereoselektiven Hydrolyse eines der beiden Stereoisomere anfallenden optisch aktiven Verbindungen und/oder das nicht hydrolysierte Esterenantiomer aus dem Reaktionsmedium gewonnen wird. Die Butinol I Esterase kann aber auch solche Ester der Formel I hydrolysieren, die nicht optisch aktiv sind.

Gegenstand der Erfindung sind auch Verfahren zur enantioselektiven Umesterung, wobei ein Stereoisomerengemisch eines optisch aktiven Alkohols der Formel II mit einem Ester der Formel I in Gegenwart einer in den Patentansprüchen definierten Butinol I Esterase in Kontakt gebracht wird und das nicht umgesetzte Alkoholstereoisomer aus dem Reaktionsmedium gewonnen wird, oder ein Stereoisomerengemisch eines optisch aktiven Esters der Formel I mit einem Alkohol der Formel II in Gegenwart der Butinol I Esterase in Kontakt gebracht wird und ein Stereoisomer des im Ester enthaltenen optisch aktiven Alkohols aus dem Reaktionsmedium gewonnen wird. Vorzugsweise werden zur Umesterung Vinylester als Acylierungsmittel für einen optisch aktiven Alkohol verwendet. Dies ist deshalb vorteilhaft, weil die Alkoholfunktion des Vinylesters nach der Umsetzung durch Tautomerisierung nicht mehr für die Rückreaktion zur Verfügung steht. Die Butinol I Esterase katalysiert auch Umesterungsprozesse, bei denen weder der Ester noch der Alkohol optisch aktiv sind.

Bevorzugte Substrate zur Esterhydrolyse sind Ester des Ethanols, Propanols, Butanols und besonders bevorzugt Butinylester (Butinolester, Ester des 1-Methyl-prop-2-ynols) mit Carbonsäuren wie beispielsweise Essigsäure, Propansäure, Buttersäure, Pentansäure, Hexansäure, Heptansäure, Octansäure, Milchsäure, 2-Ethylhexansäure, 3-Methylbuttersäure, Methoxyessigsäure, 2-Methylpropionsäure, 2-Butensäure, 3-Chlorpropionsäure und 2-Methylpentansäure. Besonders bevorzugt sind Buttersäure- (Butinylbutyrat) und Methylbuttersäurebutinylester.

Bevorzugte Alkohole bei der Umesterung sind Ethanol, Propanol und Butanol, besonders bevorzugt ist Butinol.

Bevorzugte Ester bei der Umesterung sind Vinylester, wie beispielsweise Essigsäure-, Propionsäure- und Buttersäurevinylester.

Als Reaktionsmedium werden in oben stehenden Verfahren organische Lösungsmittel verwendet, wie beispielsweise Alkane, Ether, Toluol, Dioxan, Methylisobutylketon, Methyl-tertiär-butylether (MTBE) und dergleichen. Bei der Esterhydrolyse können auch Gemische aus der verwendeten Pufferlösung und organischen Lösungsmitteln wie beispielsweise MTBE und Heptan oder Toluol verwendet werden.

Die Racematspaltung, d.h. die Enantioselektivität, und Reaktionsgeschwindigkeit sind über Größe und Hydrophobizität des Säureteils beeinflußbar. Die Reaktion wird vorzugsweise bei Raumtemperatur bei einem pH-Wert von 6 bis 9, besonders bevorzugt bei einem pH-Wert von 7,0 bis 7,4 durchgeführt. Dabei kann die Esterase in Form von isoliertem oder aufgereinigtem Enzym, Zellen des die Esterase exprimierenden Mikroorganismus, dessen Kulturüberstand, Zelllysat oder Extrakt, oder als immobilisierte Esterase eingesetzt werden. Die Reaktionsprodukte können aus der Reaktionslösung durch dem Fachmann bekannte chemische oder physikalische Trennverfahren isoliert werden. Die Butinol I Esterase kann aus dem Reaktionsgemisch durch Membranfiltration isoliert werden.

Die Immobilisierung der Esterase kann mit Hilfe von Polyacrylamid, Alginsäure oder Carragenen erfolgen. Die Esterase läßt sich auch mittels bekannter Methoden kovalent oder durch Absorption an passende Carrier binden. Vorzugsweise wird die Butinol I Esterase durch Lyophilisation auf Kieselgur oder durch Ammoniumsulfatfällung immobilisiert.

Wie oben erwähnt ist die Butinol I Esterase aus Pseudomonas glumae Lu 2023 erhältlich. Sie läßt sich allerdings auch mittels bekannter Peptidsyntheseverfahren herstellen.

Weiterhin ist die Butinol I Esterase auch aus eukaryotischen oder prokaryotischen Organismen erhältlich, wenn diese die Butinol I Esterase exprimieren, wie beispielsweise Mikroorganismen, die einen erfindungsgemäßen Vektor tragen.

Gegenstand der Erfindung sind somit auch Verfahren zur Herstellung erfindungsgemäßer Butinol I Esterasen wobei man Mikroorganismen, welche die Butinol I Esterase produzieren, oder einen mit einem erfindungsgemäßen Vektor transformierten Mikroorganismus kultiviert, gegebenenfalls die Expression der Butinol I Esterase induziert und die Butinol I Esterase aus der Kultur isoliert. Die Mikroorganismen können nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Nach Kultivierung werden die Zellen aufgeschlossen und die Butinol I Esterase durch Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren, vorzugsweise Glaskugelmühlen, oder durch Kombination mehrerer der aufgeführten Verfahren aufgeschlossen werden. Nach einer Zentrifugation bleiben Proteine und andere lösliche Moleküle im Überstand. Durch Ausfällen der DNA mit Manganchlorid kann eine Lösung mit deutlich niedrigerer Viskosität erhalten werden. Proteine können durch "Aussalzen" beispielsweise unter Verwendung von Ammoniumsulfat oder Kaliumphosphat selektiv präzipitiert werden. Die Präzipitation kann auch durch pH- oder Temperaturveränderung oder durch organische Lösungsmittel wie Methanol, Ethanol oder Aceton erfolgen. Nach der Präzipitation durch Salze können diese wieder durch Dialyse entfernt werden.

Eine weitere Aufreinigung der Butinol I Esterase kann mit bekannten, chromatographischen Verfahren erzielt werden, wie Molekularsieb-Chromatographie (Gelfiltration), Q-Sepharose-Chromatographie, Ionenaustausch-Chromatographie und hydrophobe Chromatographie, sowie mit anderen üblichen Verfahren wie Ultrafiltration, Kristallisation und nativer Gelelektrophorese.

Die Erfindung wird durch die nachfolgenden nicht limitierenden Beispiele unter Bezugnahme auf beiliegende Figuren näher erläutert. Dabei zeigt:

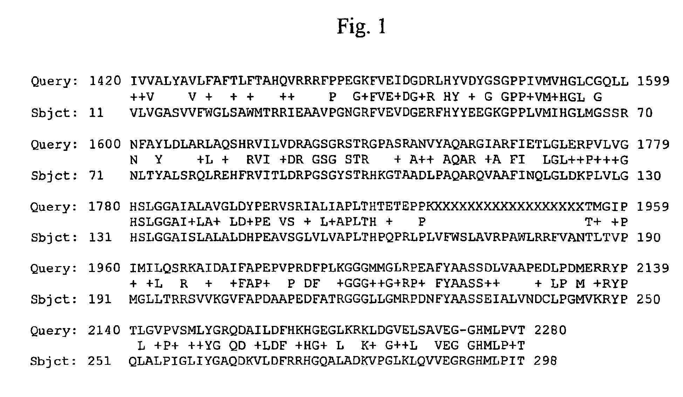

Figur 1 ein Sequenz-Alignment einer erfindungsgemäßen Aminosäure-Teilsequenz der Butinol I Esterase mit einer Teilsequenz einer Lacton-spezifischen Esterase aus Pseudomonas fluorescens ist in Fig.1 gezeichnet. Query: Teil-Sequenz des erfindungsgemäßen Klo nes LU2898. Sbjct: Teil-Sequenz des P. fluorescens Enzyms, (Accession No.: 087637)

### Beispiel 1

### Selektion einer Butinol I Esterase exprimierenden Mutante von Pseudomonas glumae

Ausgangsstamm des Screenings war der Lipase-Produzent Pseudomonas (Burkholderia) glumae Lu 8093. Mit der von diesem Stamm produzierten Lipase lassen sich eine ganze Reihe interessanter Reaktionen durchführen (Balkenhohl, F. et al., J. prakt. Chem. 339, (1997), 381-384). Milchsäureester, Methylbuttersäureester und Phenylessigsäureester sind jedoch kein Substrat für die Lipase und können auch sonst nicht von diesem Stamm hydrolysiert werden.

Die Hydrolyseprodukte sind jedoch als Kohlenstoffquelle verwertbar. Es wurde deshalb nach Mutanten von Lu 8093 gesucht, die diese Ester hydrolysieren können und mit den Hydrolyseprodukten als Kohlenstoffquelle wachsen können. Mutanten mit neuer Esteraseaktivität sollten sich daher durch Wachstum auf diesen Estern zu erkennen geben.

Selektionsbedingungen: Lu 8093 wurde auf Medium für 16 h kultiviert und durch Zentrifugation geerntet. Die Zellen wurden 2 mal mit Saline gewaschen. 10⁶ Zellen wurden auf Minimalmedium Platten, die 0,5 bzw. 1,0 g/l Ethylphenylacetat als einzige Kohlenstoffquelle enthielten, ausplattiert. Zunächst zeigte sich jedoch kein Wachstum. Erst nach 4 bis 6 Tagen waren einzelne Kolonien zu erkennen. Deren Zahl nahm in den nächsten Tagen weiter zu.

Von den so erhaltenen Esterase-positiven Mutanten wurde die Mutante Lu 2023 ausgewählt. Überraschenderweise war die neue Esterase-Aktivität auch zur selektiven Hydrolyse kleiner organischer Moleküle geeignet. Am Beispiel von Butinolester wurde die selektive Hydrolyse gezeigt.

### Beispiel 2

### Fermentation von Pseudomonas glumae Lu 2023

Zur Gewinnung der Butinol I Esterase wurde Pseudomonas glumae Lu 2023 im 14 1-Maßstab kultiviert und die aktive Biomasse geerntet.

Im Labor wurde Pseudomonas glumae Lu 2023 auf Agarplatten mit Mineralsalzmedium M12 mit 1 g/l EPA ausgestrichen und bei 28°C für 36 bis 48 Stunden inkubiert. Die Platten konnten dann bei 4°C vier Wochen gelagert werden.

Die Fermentation des Stammes wurde in einem 14 1-Fermenter Infors xxy durchgeführt. Für die Vorkultur wurden 250 ml Medium mit 2 bis 3 Pt-Ösen beimpft und 24 h bei 200 Upm und 28°C inkubiert. Die Hauptkultur wurde unter folgenden Bedingungen durchgeführt:
Temperatur 28°C

| | |
|---|---|
| Zuluft | 7 l/min |
| Rührung | 600 Upm |

Fermentationslaufzeit etwa 24 h
Eingebaute pH- und pO₂-Messung
Medium für Vorkultur und Hauptkultur

| | |
|---|---|
| 15 g/l | Springer Hefeautolysat 65% |
| 1,6 g/l | Magnesiumsulfat x 7 Wasser |
| 0,02 g/l | Calciumchlorid x 2 Wasser |
| 3,5 g/l | Kaliumdihydrogenphosphat |
| 3,5 g/l | Dikaliumhydrogenphosphat |
| 5 g/l | di-Ammoniumhydrogenphosphat |
| 6 ml | Pluriol P2000 Antischaummittel |

Die obenstehenden Inhaltsstoffe wurden in VE-Wasser gelöst und mit 25% Ammoniaklösung den pH auf 6,5 eingestellt. 5 ml/l Trace Elements (Spurensalzlsg.) und 2 g/l Glucose wurden separt sterilfiltriert.

Nach dem Sterilisieren und Komplettieren des Mediums wurden 0,5 g/l Ethylphenylacetat in den Fermenter gegeben. Während der Fermentation auftretender Schaum wurde durch die Zugabe von Pluriol P2000 eingedämmt. Die Fermentation wurde abgebrochen, wenn der pO₂-Wert im Fermenter wieder über 85% anstieg. Der Fermenterinhalt wurde dann bei einer Temperatur von unter 15°C und etwa 9000 bis 10000 g zentrifugiert und der Klarlauf verworfen. Die Zellmasse wurde bei -16°C eingefroren.

### Beispiel 3

### Reinigung der Butinol I Esterase aus Pseudomonas glumae Lu 2023

Zellen (100 ml, 50 g Feuchtmasse) von Pseudomonas glumae (Lu 2023) wurden in einer Glaskugelmühle (100 ml Glaskugeln, Durchmesser 0,5 mm) bei 4°C und 3000 Upm aufgeschlossen. Nach Zentrifugation (30 min, 10000 rpm) und Waschen der Glaskugeln wurde mit dem Überstand (300 ml) eine Manganchloridfällung (pH 7 bis 7,5; 50 mM Endkonzentration) durchgeführt. Nach erneuter Zentrifugation wurde der pH des Überstandes auf 8,0 eingestellt und EDTA zu einer Konzentration von 50 mM zugegeben. Dieses Volumen wurde durch Chromatographie an Q-Sepharose (300 ml) gereinigt. Nach der Aufgabe der Probe wurde die Säule mit 50 mM Tris/HCl gespült. Die Wertfraktion wurde gesammelt und durch Ulträfiltration (100 kDa) konzentriert. Durch Molekularsiebchromatographie (5 cm Durchmesser, 90 cm Höhe; S-300 Material) wurde die Butinol I Esterase von einer unspezifischen Esterase getrennt. Die erhaltene aktive Fraktion war trübe und wurde erneut konzentriert. Die Esterase war offensichtlich membrangebunden. Die Membranfraktion wurde nun zunächst mit einer Protease verdaut (Trypsin, Gewichtsverhältnis 1:50 bis 1:100). Dabei verschwanden alle Proteine aus der Membranfraktion bis auf wenige Banden in der SDS-Polyacrylamidgelelektrophorese. Die Aktivität blieb erhalten. Diese Banden wurden durch eine native Gelelektrophorese (0,04% SDS) voneinander getrennt und ein Aktivitätstest in diesem nativen Gel identifizierte die Esterase. Diese wurde aus dem Gel eluiert und zeigte sich nun als saubere Bande in einer denaturierenden SDS-Polyacrylamidgelelektrophorese.

Das so gereinigte Protein wurde auf eine PVDF-Membran durch Blotten überführt und sequenziert oder die Peptide wurden nach Trypsinspaltung durch Reversed-Phase HPLC getrennt und sequenziert. Da der Aminoterminus des Proteins blockiert war, wurden nur tryptische Peptide erhalten. Die verschiedenen Aminosäuresequenzen wiesen schwache Homologien zu einer Muconatcycloisomerase, EC 5.5.1.1, aus Acinetobacter Iwoffii und Pseudomonas putida, sowie zur Lactonesterase aus Pseudomonas fluoreszens auf. Das Peptid mit der Sequenz AIDAIFAPEGV wies Homologie zu Pectinesterasen (EC 3.1.1.11) auf.

Ein Sequenz-Alignment einer erfindungsgemäßen Aminosäure-Teilsequenz mit einer Teilsequenz einer Lacton-spezifischen Esterase aus Pseudomonas fluorescens ist in Fig.1 gezeichnet.

### Beispiel 4

### Immobilisierung der Butinol I Esterase

Zur Immobilisierung wurden verschiedene Methoden eingesetzt.
1. Die Butinol I Esterase wurde durch Fällung mit Aceton in Gegenwart von Kieselgur weitgehend inaktiviert. 25 mg Protein wurde mit 3,5 g Kieselgur (Merck) versetzt und 1,4 1 Aceton (-20°C) wurde für 10 min zugegeben. Der beladene Träger wurde dann über eine G3 Glasnutsche abgetrennt, der Filterkuchen mit kaltem Aceton gewaschen und getrocknet.
2. Die Butinol I Esterase bindet nicht an Accurel (Akzo).
3. Die Butinol I Esterase konnte durch (2,3 Units/g, EPA-Test) Lyophilisation auf Kieselgur immobilisiert werden. Dazu wurde die Enzymlösung mit Kieselgur vermischt und bei -80°C gefroren. Anschließend wurde der Feststoff durch Lyophilisation getrocknet.
4. Die Butinol I Esterase wurde (454 mUnits/g, EPA-Test) durch Ammoniumsulfatfällung immobilisiert. Dazu wurde das Enzym mit einer Sättigung von 80% an Ammoniumsulfat in Gegenwart von Kieselgur gefällt.

### Beispiel 5

### Racematspaltung mit der Butinol I Esterase aus Pseudomonas glumae Lu 2023

### Durchführung (Standardansatz)

100 Units Butinol I Esterase wurden mit 20 mMol Butinol-butyrat (Buttersäure-1-methyl-prop-2-ynyl-ester) in Phosphatpuffer (200 ml, 10 mmolar, pH 7,4) unter Rühren umgesetzt. Der pH-Wert wurde kontinuierlich gemessen und durch Zugabe von Natronlauge auf ca. pH 7,4 gehalten. Zu den in der Tabelle 1 angegebenen Zeiten wurden Proben entnommen, mit Methyl-tertiär-butylether (MTBE) zweimal ausgeschüttelt und die organische Phase durch GC (Chiraldex GTA) analysiert. Die Butinol I Esterase konnte durch Membranfiltration aus dem Reaktionsgemisch entfernt werden.

Mit steigender Anreicherung des weniger bevorzugten Esterenantiomers, wurde dieses in steigendem Maße umgesetzt. Dies führte nach etwa 45 Minuten zu einem Absinken des ee-Wertes von S-Butinol im Reaktionsgemisch. Der ee-Wert des Produkts erreichte sein Maximum nach ca. 30 bis 40 min mit 84% (83-97,9%). Der ee-Wert des Eduktes stieg im Verlauf von 90 Minuten auf über 99%. Der ee-Wert (Enantiomerenüberschuß) ist definiert als die Menge des bevorzugt umgesetzten Enantiomers in Prozent abzüglich der Menge des weniger bevorzugt umgesetzten Enantiomers in Prozent. Dieser Wert entspricht in den meisten Fällen der optischen Reinheit. Der pH-Abfall bis zum Zeitpunkt 30 Minuten verlief linear. Ab ca. 100 Minuten war die pH-Wert Änderung vernachlässigbar.

Die Restaktivität der Esterase in der wässrigen Phase betrug nach dem Ausschütteln noch ca 50%.

**Tabelle 1**

| Zeit | ee-Wert Produkt (S)-Butinol | ee-Wert Edukt (R)-Butinolester | Umsatz Ester in % (korr.) |
|---|---|---|---|
| 0 min | nd | 5,20 | nd |
| 7 min | nd | 10,20 | nd |
| 13 min | 75,50 | 20,40 | 12 |
| 20 min | 81,80 | 29,10 | 16 |
| 26 min | 83,90 | 42,00 | 22 |
| 32 min | 84,60 | 53,70 | 27 |
| 45 min | 84,00 | 78,80 | 36 |
| 70 min | 70,80 | 97,10 | 47 |
| 90 min | 69,60 | 99,10 | 52 |
| 121 min | 63,10 | 99,40 | 56 |
| 150 min | 52,00 | 99,50 | 67 |

Tabelle 1 zeigt den Enantiomerenüberschuß bei der Umsetzung von Butinol-butyrat mit der Butinol I Esterase in Abhängigkeit von der Zeit. R- und S-Konfiguration definieren, nach der R/S-Nomenklatur von Cahn, Prelog und Ingold, die beiden Enantiomeren eines chiralen Moleküls. Der Umsatz ist der Anteil des umgesetzten Esters im Reaktionsgemisch.

### Beispiel 6

### Abhängigkeit der Spezifität der Butinol I Esterase von der Größe und Hydrophobizität/Ladung des Säureteils des Esters

### Standardansatz

100 Units Butinol I Esterase wurden mit 20 mMol Butinolester in Phosphatpuffer (200 ml, 10 mmolar, pH 7,4) unter Rühren umgesetzt. Der pH-Wert wurde kontinuierlich gemessen und durch kontinuierliche Titration auf pH 7,0 gehalten. Entnommene Proben wurden mit Methyl-tertiär-butylether (MTBE) zweimal ausgeschüttelt und die organische Phase durch GC (Chiraldex GTA) analysiert.

### Ergebnis

Die Qualität der Racematspaltung und die Reaktionsgeschwindigkeit waren von Größe und Hydrophobizität des Säureteils abhängig. Die besten Substrate für die Butinolesterase waren die Buttersäure- und Methylbuttersäure-butinolester. Lipasen sind gegenüber diesen Substraten inaktiv. Dies gilt auch für langkettige Ester wie n-Decansäurebutinylester.

**Tabelle 2**

| Säurekomponente | ee-Wert [%] | Umsatz [%] | E |
|---|---|---|---|
| Acetat | 73 (S) | 48 | 12 |
| Butyrat | 95 (S) | 36 | 67 |
| Pentanoat | 74 (S) | 47 | 13 |
| Hexanoat | 66 (S) | 44 | 8 |
| Octanoat | 64 (S) | 43 | 8 |
| 2-Ethyl-hexanoat | kein Umsatz | | |
| Phenylacetat | 51 (S) | 12 | 3 |
| 3-Phenylpropionat | 73 (S) | 44 | 11 |
| 3-Cyclohexylpropionat | 22 (S) | 18 | 2 |

Tabelle 2 zeigt den Enantiomerenüberschuß bei der Umsetzung von Estern mit der Butinol I Esterase in Abhängigkeit vom Säureteil des umgesetzten Esters

### Beispiel 7

### Umesterung in organischem Medium unter Verwendung der Butinol I Esterase

10 mmol rac.-Butinol und 5 mmol Buttersäurevinylester wurden in 50 ml Methyl-tertiär-butylether (MTBE) gelöst und mit 9 Units Butinol I Esterase (3,3 g) geträgert auf Kieselgur versetzt und für 24 h bei RT geschüttelt. Nach Filtration wurde das Lösungsmittel entfernt und das Produktgemisch per GC charakterisiert.

Zurück blieb bei 47% Umsatz (R)-Butinol (18% ee) und das Butyrat des (S)-Butinols (45% ee).

In Methylisobutylketon wurde bei 43% Umsatz (R)-Butinol mit 16% ee und das Butyrat des (S)-Butinols mit 52% ee erhalten.

Tabelle 3 zeigt den Enantiomerenüberschuß bei der Umsetzung von Estern mit der Butinol I Esterase in Abhängigkeit vom Säureteil des umgesetzten Esters.

**Tabelle 3**

| Ansatz Nr. | Edukt | pH-Wert | Temp. [°C] | Puffersystem Lsg. [mmol/l] | Zusatzstoffe | ee-Werte¹⁾ |
|---|---|---|---|---|---|---|
| 8 | n-Decansäurebutinylester | 7,0 | RT | Phosphat 10 | keine | 54,37 |
| 14 | n-Pentansäurebutinylester | 7,0 | RT | Phosphat 10 | keine | 80,40 |
| 15 | 2-Ethylhexansäurebutinylester | 7,0 | RT | Phosphat 10 | keine | 81,77 |
| 16 | Butinylbutyrat | 7,0 | RT | Phosphat 10 | keine | 83,90 |
| 17 | Butinylbutyrat | 7,0 | RT | Phosphat 10 | 0,5% Triton | 80,83 |
| 18 | n-Hexansäurebutinylester | 7,0 | RT | Phosphat 10 | 0,5% Triton | 78,63 |
| 19 | n-Octansäurebutinylester | 7,0 | RT | Phosphat 10 | 0,5% Triton | 74,70 |
| 20 | Butinylbutyrat | 7,0 | RT | Phosphat 10 | 10% n-Propanol | 87,47 |
| 21 | Butinylbutyrat | 7,0 | RT | Phosphat 10 | 1 M NaCl | 85,70 |
| 23 | n-Pentansäurebutinylester | 7,0 | RT | Phosphat 10 | 0,5% Triton | 84,40 |
| 24 | Butinylbutyrat | 6,0 | RT | Phosphat 10 | keine | 85,37 |
| 25 | Butinylbutyrat | 8,0 | RT | Tris 10 | keine | 85,33 |
| 26 | Butinylbutyrat | 7,0 | 10 | Phosphat 10 | keine | 85,90 |
| 27 | Butinylbutyrat | 7,0 | 37 | Phosphat 10 | keine | 75,67 |
| 28 | 3-Methylbuttersäurebutinylester | 7,0 | RT | Phosphat 10 | keine | 90,50 |
| 29 | Methoxyessigsäurebutinylester | 7,0 | RT | Phosphat 10 | keine | 76,33 |
| 31 | Butinylbutyrat | 7,0 | RT | Phosphat 10 | keine | 85,00 |
| 32 | Butinylbutyrat | 7,0 | RT | Phosphat 10 | 2-Phasensystem | 84,93 |
| 33 | 3-Methylbuttersäurebutinylester | 7,0 | RT | Phosphat 10 | 2-Phasensystem | 92,70 |
| 34 | 2-Methylpropionsäurebutinylester | 7,0 | RT | Phosphat 10 | keine | 89,17 |
| 35 | 2-Butensäurebutinylester | 7,0 | RT | Phosphat 10 | keine | 76,03 |
| 36 | 3-Chlorpropionsäurebutinylester | 7,0 | RT | Phosphat 10 | keine | 71,13 |
| 40 | 2-Methylpentansäurebutinylester | 7,0 | RT | Phosphat 10 | keine | 85,93 |

| | | | | | | |
|---|---|---|---|---|---|---|
| ¹⁾ Mittelwerte der 3 besten ee-Werte von S-Butinol | | | | | | |

## Patentansprüche

1. Protein mit Butinol-I-Esterase-Aktivität, umfassend wenigstens eine Aminosäure-Teilsequenz gemäß SEQ ID NO: 3, 4, 5 oder 6, welches die Befähigung zur enantioselektiven Hydrolyse von Carbonsäurebutinylestern aufweist.

2. Protein mit Butinol-I-Esterase-Aktivität, umfassend eine Aminosäuresequenz gemäß SEQ ID NO:2, oder eine dazu homologe Aminosäuresequenz mit einem Homologiegrad von wenigstens 60%, berechnet nach dem Algorithmus von Pearson und Lipman, Proc. Natl. Acad. Sci (USA) 85 (8), 1988, 2444-2448, welches die Befähigung zur enantioselektiven Hydrolyse von Carbonsäurebutinylestern aufweist.

3. Protein gemäß einem der vorhergehenden Ansprüche, umfassend eine Polypeptidkette mit einem Molekulargewicht von etwa 41300 Da.

4. Protein gemäß einem der vorhergehenden Ansprüche, **dadurch gekennzeichnet, dass** es aus Pseudomonas glumae Lu 2023 mit der Hinterlegungsnummer DSM 13176 erhältlich ist.

5. Protein gemäß einem der vorhergehenden Ansprüche, **dadurch gekennzeichnet, dass** es zusätzlich wenigstens eine der folgenden Reaktionen katalysiert:
a) enantioselektive Hydrolyse optisch aktiver Ester der Formel I
R¹-COO-R² (I),
worin
R¹ für geradkettiges oder verzweigtes, gegebenenfalls ein- oder mehrfach substituiertes C₁-C₁₀-Alkyl, C₂-C₁₀-Alkenyl, C₂-C₁₀-Alkinyl und R² für geradkettiges oder verzweigtes, gegebenenfalls ein- oder mehrfach substituiertes C₁-C₁₀-Alkyl, C₂-C₁₀-Alkenyl, C₂-C₁₀-Alkinyl, C₇-C₁₅-Aralkyl oder für einen ein- oder mehrkernigen, gegebenenfalls ein- oder mehrfach substituierten aromatischen Rest steht,
R¹ und/oder R² wenigstens ein asymmetrisches Kohlenstoffatom umfasst; und
b) enantioselektive Umesterung eines Esters der Formel I mit einem optisch aktiven Alkohol der Formel II
R²-OH (II),
worin R² eine der obenstehenden Bedeutungen besitzt, und gegebenenfalls wenigstens ein asymmetrisches Kohlenstoffatom aufweist.

6. Polynukleotid, das für ein Protein gemäß einem der vorhergehenden Ansprüche kodiert, und die damit unter stringenten Bedingungen hybridisierenden oder dazu komplementären Sequenzen, jeweils umfassend eine Nukleotidteilsequenz von wenigstens 30 aufeinanderfolgenden Nukleotidresten einer Nukleinsäuresequenz gemäß SEQ ID NO:1 oder eine dazu komplementäre Nukleotoidteilsequenz von wenigstens 30 aufeinanderfolgenden Nukleotidresten.

7. Expressionskassette, umfassend wenigstens ein Polynukleotid gemäß Anspruch 6 operativ verknüpft mit wenigstens einer regulatorischen Nukleinsäuresequenz.

8. Rekombinanter Vektor zur Transformation eines eukaryontischen oder prokaryontischen Wirts, umfassend ein Polynukleotid gemäß Anspruch 6, oder eine Expressionskassette gemäß Anspruch 7.

9. Verfahren zur Herstellung eines Proteins gemäß einem der Ansprüche 1 bis 5, wobei man einen Mikroorganismus, welcher das Protein endogen produziert, oder einen mit einem Vektor gemäß Anspruch 8 transformierten Mikroorganismus kultiviert und das Protein aus der Kultur isoliert.

10. Verfahren gemäß Anspruch 9, wobei der Mikroorganismus Pseudomonas glumae Lu 2023 mit der Hinterlegungsnummer DSM 13176 ist.

11. Protein, erhältlich nach einem Verfahren gemäß einem der Ansprüche 9 oder 10, umfassend wenigstens eine Aminosäure-Teilsequenz gemäß SEQ ID NO: 3, 4, 5 oder 6,das zur enantioselektiven Hydrolyse von Carbonsäurebutinylestern befähigt ist.

12. Pseudomonas glumae Lu 2023 mit der Hinterlegungsnummer DSM 13176.

13. Mikroorganismus, **dadurch gekennzeichnet, dass** er einen Vektor gemäß Anspruch 8 trägt.

14. Verfahren zur enantioselektiven Esterhydrolyse unter Verwendung eines Proteins gemäß einem der Ansprüche 1 bis 5, wobei
a) das Protein mit einem Stereoisomerengemisch eines optisch aktiven Esters der Formel I in Kontakt gebracht wird; und
b) die bei der stereoselektiven Hydrolyse eines der Stereoisomere anfallenden optisch aktiven Verbindungen und/oder das nicht hydrolysierte Esterenantiomer aus dem Reaktionsmedium gewonnen wird.

15. Verfahren zur enantioselektiven Umesterung, wobei
a) ein Stereoisomerengemisch eines optisch aktiven Alkohols der Formel II mit einem Ester der Formel I in Gegenwart eines Proteins gemäß einem der Ansprüche 1 bis 5 in Kontakt gebracht wird und das nicht umgesetzte Alkoholstereoisomer aus dem Reaktionsmedium gewonnen wird; oder
b) ein Stereoisomerengemisch eines optisch aktiven Esters der Formel I mit einem Alkohol der Formel II in Gegenwart eines Proteins gemäß einem der Ansprüche 1 bis 5 in Kontakt gebracht wird und ein Stereoisomer des im Ester enthaltenen optisch aktiven Alkohols aus dem Reaktionsmedium gewonnen wird.

16. Verfahren nach Anspruch 15, wobei man zur Umesterung einen Vinylester als Acylierungsmittel für einen optisch aktiven Alkohol verwendet.

17. Verfahren gemäß einem der Ansprüche 14, 15 oder 16, wobei als Reaktionsmedium ein organisches Lösungsmittel verwendet wird.

## Claims

1. A protein having butynol I esterase activity, which comprises at least one amino acid part sequence according to SEQ ID NO.: 3, 4, 5 or 6, and which has the capability of enantioselectively hydrolysing butynyl carboxylates.

2. A protein having butynol I esterase activity, which comprises an amino acid sequence according to SEQ ID NO: 2, or an amino acid sequence homologous thereto and with a degree of homology of at least 60% as calculated according to the algorithm by Pearson and Lipman, Proc. Natl. Acad. Sci (USA) 85 (8), 1988, 2444-2448, and which has the capability of enantioselectively hydrolysing butynyl carboxylates.

3. The protein according to either of the preceding claims, which comprises a polypeptide chain having a molecular weight of about 41 300 Da.

4. The protein according to any of the preceding claims, wherein said protein is obtainable from Pseudomonas glumae Lu 2023 with deposition number DSM 13176.

5. The protein according to any of the preceding claims, wherein said protein additionally catalyzes at least one of the following reactions:
a) enantioselective hydrolysis of optically active esters of the formula I
R¹-COO-R² (I),
in which
R¹ is a straight-chain or branched, optionally monosubstituted or polysubstituted C₁-C₁₀-alkyl, C₂-C₁₀-alkenyl, C₂-C₁₀-alkynyl and R² is a straight-chain or branched, optionally monosubstituted or polysubstituted C₁-C₁₀-alkyl, C₂-C₁₀-alkenyl, C₂-C₁₀-alkynyl, C₇-C₁₅-aralkyl or a mononuclear or polynuclear, optionally monosubstituted or polysubstituted aromatic radical,
R¹ and/or R² comprise at least one asymmetric carbon; and
b) enantioselective transesterification of an ester of the formula I with an optically active alcohol of the formula II
R²-OH (II),
in which R² has one of the above meanings and, optionally, has at least one asymmetric carbon.

6. A polynucleotide, which codes for a protein according to any of the preceding claims, and the sequences hybridizing therewith under stringent conditions or complementary thereto, in each case comprising a nucleotide part sequence of at least 30 consecutive nucleotide residues of a nucleic acid sequence according to SEQ ID NO: 1 or a nucleotide part sequence complementary thereto of at least 30 consecutive nucleotide residues.

7. An expression cassette, which comprises at least one polynucleotide according to claim 6 which is operatively linked to at least one regulatory nucleic acid sequence.

8. A recombinant vector for transforming a eukaryotic or prokaryotic host, which comprises a polynucleotide according to claim 6, or an expression cassette according to claim 7.

9. A method for preparing a protein according to any of claims 1 to 5, wherein a microorganism which produces the protein endogenously or a microorganism transformed with a vector according to claim 8 is cultured and the protein is isolated from the culture.

10. The method according to claim 9, wherein the microorganism is Pseudomonas glumae Lu 2023 with deposition number DSM 13176.

11. A protein obtainable according to a method according to claim 9 or 10, which protein comprises at least one amino acid part sequence according to SEQ ID NO: 3, 4, 5 or 6, and which is capable of enantioselectively hydrolysing butynyl carboxylates.

12. A Pseudomonas glumae Lu 2023 with deposition number DSM 13176.

13. A microorganism, which comprises a vector according to claim 8.

14. A method for enantioselective ester hydrolysis using a protein according to any of claims 1 to 5, wherein
a) the protein is contacted with a stereoisomer mixture of an optically active ester of the formula I; and
b) the optically active compounds arising from the stereoselective hydrolysis of any of the stereoisomers and/or the non-hydrolyzed ester enantiomer are obtained from the reaction medium.

15. A method for enantioselective transesterification, wherein
a) a stereoisomer mixture of an optically active alcohol of the formula II is contacted with an ester of the formula I in the presence of a protein according to any of claims 1 to 5 and the unreacted alcohol stereoisomer is obtained from the reaction medium; or
b) a stereoisomer mixture of an optically active ester of the formula I is contacted with an alcohol of the formula II in the presence of a protein according to any of claims 1 to 5 and a stereoisomer of the optically active alcohol contained in the ester is obtained from the reaction medium.

16. The method according to claim 15, wherein a vinyl ester is used for transesterification as acylating agent for an optically active alcohol.

17. The method according to any of claims 14, 15 and 16, wherein the reaction medium used is an organic solvent.

## Revendications

1. Protéine présentant une activité de butynol-I-estérase comprenant au moins une séquence partielle d'acides aminés selon les SEQ ID No : 3, 4, 5 ou 6, qui présente l'aptitude à l'hydrolyse énantiosélective d'esters butynyliques d'acides carboxyliques.

2. Protéine présentant une activité de butynol-I-estérase, comprenant une séquence d'acides aminés selon la SEQ ID No : 2, ou une séquence d'acides aminés homologue à celle-ci, présentant un degré d'homologie d'au moins 60%, calculé selon l'algorithme de Pearson et Lipman, Proc. Natl. Acad. Sci (USA) 85 (8), 1988, 2444-2448, qui présente l'aptitude à l'hydrolyse énantiosélective d'esters butynyliques d'acides carboxyliques.

3. Protéine selon l'une quelconque des revendications précédentes, comprenant une chaîne polypeptidique présentant un poids moléculaire d'environ 41300 Da.

4. Protéine selon l'une quelconque des revendications précédentes, **caractérisée en ce qu'**elle peut être obtenue à partir de Pseudomonas glumae Lu 2023 présentant le numéro de dépôt DSM 13176.

5. Protéine selon l'une quelconque des revendications précédentes, **caractérisée en ce qu'**elle catalyse en outre au moins une des réactions suivantes :
a) l'hydrolyse énantiosélective d'esters optiquement actifs de formule I
R¹-COO-R² (I)
où
R¹ représente C₁-C₁₀-alkyle, C₂-C₁₀-alcényle, C₂-C₁₀-alcynyle linéaire ou ramifié, le cas échéant monosubstitué ou polysubstitué, et R² représente C₁-C₁₀-alkyle, C₂-C₁₀-alcényle, C₂-C₁₀-alcynyle, C₇-C₁₅-aralkyle linéaire ou ramifié, le cas échéant monosubstitué ou polysubstitué ou un radical aromatique à un ou plusieurs noyaux, le cas échéant monosubstitué ou polysubstitué,
R¹ et/ou R² comprend au moins un atome de carbone asymétrique ; et
b) la transestérification énantiosélective d'un ester de formule I avec un alcool optiquement actif de formule II
R²-OH (II),
où
R² présente une des significations indiquées ci-dessus et présente le cas échéant au moins un atome de carbone asymétrique.

6. Polynucléotide qui code pour une protéine selon l'une quelconque des revendications précédentes et les séquences qui hybrident avec celui-ci dans des conditions rigoureuses ou les séquences complémentaires à celui-ci, comprenant à chaque fois une séquence partielle de nucléotides d'au moins 30 radicaux nucléotidiques successifs d'une séquence d'acides nucléiques selon la SEQ ID No : 1 ou une séquence partielle de nucléotides complémentaire à celle-ci d'au moins 30 radicaux nucléotidiques successifs.

7. Cassette d'expression, comprenant au moins un polynucléotide selon la revendication 6 lié de manière opérationnelle avec au moins une séquence d'acides nucléiques régulatrice.

8. Vecteur recombinant pour la transformation d'un hôte eucaryote ou procaryote, comprenant un polynucléotide selon la revendication 6 ou une cassette d'expression selon la revendication 7.

9. Procédé pour la préparation d'une protéine selon l'une quelconque des revendications 1 à 5, où on cultive un microorganisme, qui produit la protéine de manière endogène ou un microorganisme transformé avec un vecteur selon la revendication 8 et on isole la protéine de la culture.

10. Procédé selon la revendication 9, où le microorganisme est le Pseudomonas glumae Lu 2023 présentant le numéro de dépôt DSM 13176.

11. Protéine pouvant être obtenue selon un procédé selon l'une quelconque des revendications 9 ou 10, comprenant au moins une séquence partielle d'acides aminés selon les SEQ ID No : 3, 4, 5 ou 6, qui est apte à l'hydrolyse énantiosélective d'esters butynyliques d'acides carboxyliques.

12. Pseudomonas glumae Lu 2023 présentant le numéro de dépôt DSM 13176.

13. Microorganisme, **caractérisé en ce qu'**il porte un vecteur selon la revendication 8.

14. Procédé pour l'hydrolyse énantiosélective d'esters utilisant une protéine selon l'une quelconque des revendications 1 à 5, où
a) la protéine est mise en contact avec un mélange de stéréo-isomères d'un ester optiquement actif de formule I ; et
b) les composés optiquement actifs produits lors de l'hydrolyse stéréosélective d'un des stéréo-isomères et/ou l'énantiomère d'ester non hydrolysé est/sont obtenu(s) à partir du mélange réactionnel.

15. Procédé de transestérification énantiosélective, où
a) on met en contact un mélange de stéréo-isomères d'un alcool optiquement actif de formule II avec un ester de formule I en présence d'une protéine selon l'une quelconque des revendications 1 à 5 et le stéréo-isomère alcoolique non transformé est obtenu à partir du mélange réactionnel ; ou
b) on met en contact un mélange de stéréo-isomères d'un ester optiquement actif de formule I avec un alcool de formule II en présence d'une protéine selon l'une quelconque des revendications 1 à 5 et un stéréo-isomère de l'alcool optiquement actif contenu dans l'ester est obtenu à partir du mélange réactionnel.

16. Procédé selon la revendication 15, où on utilise pour le transestérification un ester de vinyle comme agent d'acylation pour un alcool optiquement actif.

17. Procédé selon l'une quelconque des revendications 14, 15 ou 16, où on utilise comme mélange réactionnel un solvant organique.
